# EUROPÄISCHE PATENTSCHRIFT

(11) **EP 2 666 495 B1**
(45) Veröffentlichungstag und Bekanntmachung des Hinweises auf die Patenterteilung: **12.07.2017**
(21) Anmeldenummer: 13168077.9
(22) Anmeldetag: 16.05.2013
(51) Int. Cl.: A61M 5/14, F16L 3/00, A61M 1/16

(54) **Schlauchführungselement; medizinisches Gerät zur extrakorporalen Blutbehandlung mit Schlauchführungselement**
Flexible tube guidance element; medical device for extracorporeal blood treatment with flexible tube guidance element
Elément de guidage de tuyau flexible; appareil médical pour le traitement extracorporel du sang avec élément de guidage de tuyau

(30) Priorität: 23.05.2012 DE 102012104463
(43) Veröffentlichungstag der Anmeldung: 27.11.2013
(73) Patentinhaber: B. Braun Avitum AG, 34212 Melsungen (DE)
(72) Erfinder: Schade, Andreas, 36199 Rotenburg (DE)
(74) Vertreter: Winter, Brandl, Fürniss, Hübner, Röss, Kaiser, Polte - Partnerschaft mbB

(56) Entgegenhaltungen:
- WO-A1-2009/145799
- GB-A- 2 351 523
- US-A1- 2006 237 597
- US-A1- 2009 019 678

## Beschreibung

Die Erfindung betrifft ein Schlauchführungselement, das dafür angepasst ist, einen Schlauch am Gehäuse eines medizinischen Geräts zur extrakorporalen Blutbehandlung anzubringen und zu führen.

Die Erfindung betrifft ferner ein medizinisches Gerät zur extrakorporalen Blutbehandlung mit einem solchen Schlauchführungselement.

Bei medizintechnischen Geräten zur extrakorporalen Blutbehandlung wird über Schläuche und Kammern ein extrakorporaler Blutkreislauf aufgebaut. In der Regel wird hierzu ein Einmalartikel verwendet, welcher vom Bediener am Gerät befestigt wird. Damit der Bediener die Befestigung in der vorgesehenen Art und Weise vornehmen kann, sind entsprechende Haltevorrichtungen am Gerät angebracht.

Um den Einmalartikel platzsparend am Gerät anbringen zu können, kann es erforderlich sein, den Schlauch bogenförmig zu verlegen. Dies birgt jedoch das Risiko, dass der minimale Biegeradius des Schlauchs unterschritten wird und der Schlauch abknickt. Durch die an der Knickstelle entstehenden hohen Flussgeschwindigkeiten wirken Scherkräfte, welche das Blut schädigen können. Im schlimmsten Fall wird Hämolyse erzeugt. Insbesondere sind diejenigen Schlauchstrecken betroffen, auf welche der Patient oder gegebenenfalls Personen im Umkreis des Patienten versehentlich Einfluss nehmen können. Dies ist im Wesentlichen die arterielle Leitung vom Patienten bis zur Blutpumpe des Geräts bzw. die venöse Leitung zum Patienten, so dass für diese Schlauchabschnitte Halterungen vorgesehen sein sollten, mit welchen der Bediener die Leitungen sicher in einer bestimmten Orientierung fixieren kann.

Je nach Therapieart muss der Einmalartikel jedoch auch unterschiedlich am Gerät geführt und befestigt werden. Dies bedeutet, dass therapieabhängig verschiedene Befestigungselemente für den Einmalartikel vorgesehen sein müssen und dass der Bediener wissen muss, für welche Therapieart der Schlauch auf welchem Verlegeweg angebracht wird. Die WO 2009/145799 A1 zeigt ein Schlauchführungselement, welches die Merkmale des Oberbegriffs des Anspruchs 1 aufweist. Aufgabe der Erfindung ist es daher, ein Schlauchführungselement bereitzustellen, mit dem ein Schlauch für wenigstens zwei verschiedene Verlegewege sicher an einem medizinischen Gerät angebracht werden kann, wobei insbesondere ein Abknicken des Schlauchs durch Zugbelastung verhindert werden soll.

Aufgabe der Erfindung ist es ferner, ein medizinisches Gerät zur extrakorporalen Blutbehandlung mit einem solchen Schlauchführungselement bereitzustellen. Erfindungsgemäß wird diese Aufgabe durch ein Schlauchführungselement gemäß dem unabhängigen Anspruch 1 gelöst. Vorteilhafte Weiterbildungen des Schlauchführungselements ergeben sich aus den Unteransprüchen 2-6. Die Aufgabe wird ferner durch ein medizinisches Gerät zur extrakorporalen Blutbehandlung gemäß Anspruch 7 gelöst. Vorteilhafte Weiterbildungen des medizinischen Geräts ergeben sich aus den Unteransprüchen 8-10.

Das erfindungsgemäße Schlauchführungselement dient zur Anbringung und Führung eines Schlauchs am Gehäuse eines medizinischen Geräts zur extrakorporalen Blutbehandlung. Dabei weist das Schlauchführungselement einen vorzugsweise plattenförmigen/würfelförmigen Grundkörper auf, der über seine erste (Flach-)Seite (nachfolgend Rückseite) an dem Gehäuse anbringbar ist. Auf der hierzu gegenüberliegenden zweiten Seite bzw. Vorderseite des Grundkörpers sind zwei Klemmbacken mit jeweils einer Außenwölbung so angebracht, dass die Außenwölbungen einander zugewandt sind und zwischen den Außenwölbungen ein Klemmspalt ausgebildet ist, in welchen ein Schlauch einklemmbar ist. Dabei ist der Abstand zwischen den beiden Außenwölbungen der Klemmbacken kleiner als der Schlauchdurchmesser, so dass der Schlauch zwischen den beiden Klemmbacken eingeklemmt wird. Allerdings lässt ein Freiraum hinter den Außenwölbungen dem Schlauch vorzugsweise eine gewisse Bewegungsfreiheit. Der Begriff "Klemmen" bedeutet im Sinne dieser Erfindung somit nicht unbedingt, dass der Schlauch fest zwischen den beiden Backen gehalten wird, sondern er kann auch bis zu einem gewissen Grad beweglich gehalten werden, so dass es sich eher um eine Art Führung handeln kann.

Ferner weist der Grundkörper an einer (vorzugsweise) oberen (gemäß der Figuren), dritten Seitenfläche eine Führungsrinne auf, in welche ein Schlauch einlegbar ist, und die Kante zwischen der oberen Seitenfläche und wenigstens einer weiteren Seitenfläche des Grundkörpers ist abgerundet ausgeführt. Dabei legt die Bezeichnung "obere Seitenfläche" diejenige Seitenfläche des Grundkörpers fest, die sich bei einem Schlauchführungselement oben befindet, wenn dieses an einem medizinischen Gerät montiert ist.

Wird ein solches Schlauchführungselement an einem medizinischen Gerät zur extrakorporalen Blutbehandlung angebracht, können durch die erfindungsgemäße Ausgestaltung des Bauteils wenigstens zwei Verlegewege für einen Schlauch realisiert werden. Bei beiden Verlegewegen kann ein Abknicken des Schlauchs durch Zugbelastung verhindert werden, wodurch eine Hämolyse vermieden wird.

Der erste Verlegeweg sieht dabei vor, dass ein Schlauch in den Klammspalt zwischen den beiden Klemmbacken eingedrückt wird, wodurch er am Schlauchführungselement gehalten und gegen ein Abknicken durch Zugbelastung gesichert werden kann. Insbesondere sind dazu die Radien der abgerundeten Klemmbacken, an denen der Schlauch anliegt, entsprechend so ausgeführt, dass ein Schlauch nicht an einer scharfen Kante umgebogen wird. Die Klemmbacken können daher auch als Führungsbacken bezeichnet werden.

Der zweite Verlegeweg sieht vor, dass der Schlauch in die obere Führungsrinne eingebracht wird. Die Führungsrinne kann dabei so ausgeführt sein, dass der Schlauch in der Führungsrinne eingeklemmt werden kann, es ist jedoch auch möglich, die Führungsrinne so auszuführen, dass der Schlauch lediglich lose in die Führungsrinne eingelegt bzw. aufgelegt wird. Ferner kann die Führungsrinne auch für ein loses Auflegen eines Schlauchs auf verschiedene Arten ausgeformt sein. In einem Ausführungsbeispiel der Erfindung ist sie wenigstens durch eine Vertiefung in der oberen Seitenfläche des Grundkörpers ausgebildet. Es würde aber gegebenenfalls auch ausreichen, dass die Führungsrinne wenigstens durch die Klemmbacken gebildet ist, indem die Klemmbacken über die obere Seitenfläche des Grundkörpers hinausragen. Auch eine Kombination beider Maßnahmen ist möglich.

Wird ein Schlauch im zweiten Verlegeweg in die Führungsrinne eingelegt, kann er lose an einer der seitlichen Kanten der oberen Seitenfläche herunterhängen und zu einem weiteren Funktionsteil des medizinischen Geräts geführt werden. Die abgerundete Kante im Bereich des nach unten gebogenen Schlauchs verhindert ein Abknicken in diesem Bereich, wenn eine Zugbelastung auf den Schlauch aufgebracht wird.

Die Formgebung des Bauteils kann somit für wenigstens zwei Verlegewege das Abknicken eines Schlauchs durch Zugbelastung verhindern. Auch ein Abrutschen und Lösen des Schlauchs vom Schlauchführungselement kann verhindert werden. Ein erster Verlegeweg kann beispielsweise für eine SNCO-Therapie (Single-Needle-Cross-Over- Therapie) vorgesehen sein, während für eine DN-Therapie (Dual-Needle-Therapie) ein zweiter Verlegeweg vorteilhaft sein kann. Die semantische Gestaltung des Bauteils gibt einem Bediener dabei eine sichere Schlauchverlegung vor, wobei wenigstens zwei verschiedene Verlegewege erforderlich bzw. vorteilhaft sein können, wenn für verschiedene Therapiearten unterschiedliche Funktionsteile am Gehäuse angebracht sind und somit unterschiedliche Verlegwege ermöglichen oder sogar erzwingen. Beispielsweise kann bei einer SNCO-Therapie ein Filter am Gehäuse angebracht sein, der von dem Schlauch umgangen werden muss, während die DN-Therapie diesen Filter nicht vorsieht, so dass der Schlauch anders verlegt werden kann als bei der SNCO-Therapie.

In einem Ausführungsbeispiel der Erfindung verläuft der Klemmspalt quer zur Führungsrinne, wobei er vorzugsweise senkrecht zur Führungsrinne verläuft. Er kann jedoch auch in einem von 90° verschiedenen Winkel zur Führungsrinne verlaufen.

Von der Erfindung umfasst ist ferner ein medizinisches Gerät zur extrakorporalen Blutbehandlung, das ein Gehäuse und ein Schlauchführungselement aufweist, das an einer Seite des Gehäuses angebracht ist, wobei das Schlauchführungselement erfindungsgemäß ausgebildet ist.

In einem Ausführungsbeispiel der Erfindung ist das Schlauchführungselement so ausgebildet und am Gehäuse angebracht, dass der Klemmspalt vertikal verläuft, während die Führungsrinne an der oberen Seitenfläche des Grundkörpers horizontal verläuft. Mit dem medizinischen Gerät sind dann wenigstens zwei unterschiedliche Therapiearten durchführbar, welche verschiedene Verlegewege eines Schlauchs vorsehen, wobei der Schlauch bei einem ersten Verlegeweg durch den Klemmspalt zu führen ist, während der Schlauch für den zweiten Verlegeweg in die Führungsrinne einzulegen ist. Für eine erste Therapieart wird der Schlauch somit durch das Schlauchführungselement hindurch vertikal nach unten geführt, während ein Schlauch für eine zweite Therapieart über das Schlauchführungselement hinaus und dann nach unten geführt wird.

Beispielsweise ist das Schlauchführungselement neben einem Funktionsteil des medizinischen Geräts angebracht, aus dem ein Schlauch horizontal aus einem Schlauchanschluss herausgeführt ist. Bei dem Funktionsteil kann es sich beispielsweise um eine Blutpumpe handeln, mit der Blut durch den Schlauch gefördert wird, und der Schlauch kann Blut zu- oder abführen. Das Schlauchführungselement ist so neben diesem Funktionsteil angeordnet ist, dass die obere Seitenfläche des Grundkörpers auf Höhe des Schlauchanschlusses liegt. Der Schlauch kann in dem ersten Verlegeweg teilweise in der Führungsrinne aufliegen und dann durch den Klemmspalt bzw. Führungsspalt nach unten geführt sein, während der Schlauch in dem zweiten Verlegeweg vollständig in der Führungsrinne aufliegt und über die abgerundete Kante der oberen Seitenfläche nach unten hängt. Auf diese Weise kann der Schlauch für beide Therapiearten sicher horizontal aus einem Funktionsteil des medizinisches Geräts herausgeführt und anschließend nach unten geführt werden, ohne dass es durch Zugbelastung zu einem Abknicken des Schlauchs kommen kann.

Weitere Vorteile, Besonderheiten und zweckmäßige Weiterbildungen der Erfindung ergeben sich aus den Unteransprüchen und der nachfolgenden Darstellung bevorzugter Ausführungsbeispiele anhand der Abbildungen.

Von den Abbildungen zeigt:
- Fig. 1: eine Aufsicht auf ein Ausführungsbeispiel des erfindungsgemäßen Schlauchführungselements;
- Fig. 2: eine Seitenansicht eines Schlauchführungselements gemäß Fig. 1, welches an einem Gehäuse montiert ist;
- Fig. 3: eine Teilansicht eines medizinischen Geräts mit montiertem Schlauchführungselement und einem ersten Verlegeweg für einen Schlauch; und
- Fig. 4: eine Teilansicht eines medizinischen Geräts gemäß Fig. 3 und einem zweiten Verlegeweg für einen Schlauch.

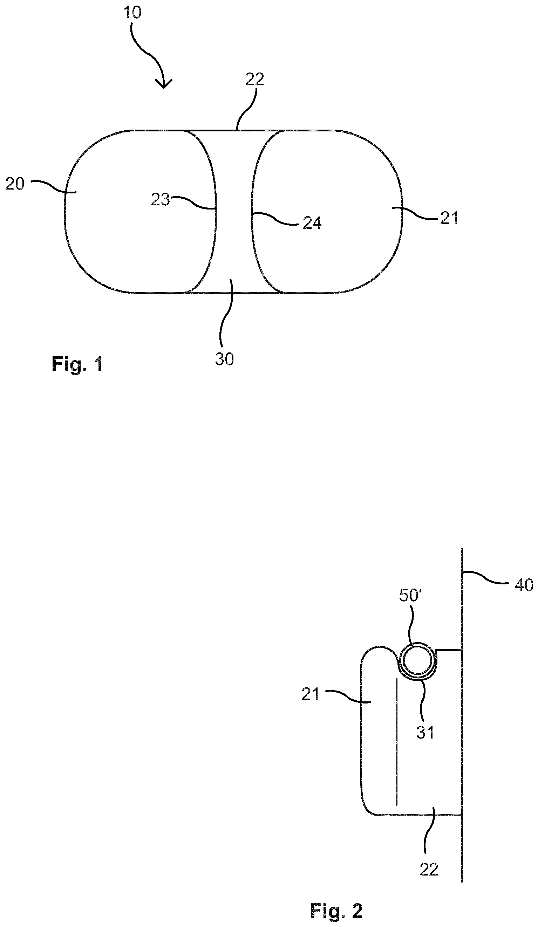

Der Aufsicht auf ein Ausführungsbeispiel des erfindungsgemäßen Schlauchführungselements gemäß Fig. 1 ist der grundsätzliche Aufbau des Schlauchführungselements 10 zu entnehmen. Es umfasst einen Grundkörper 22, welcher mit seiner Rückseite am Gehäuse eines medizinischen Geräts angebracht werden kann. Bei dem medizinischen Gerät handelt es sich insbesondere um ein Gerät zur extrakorporalen Blutbehandlung, das einen Eimalartikel verwendet, der wenigstens mehrere Schlauchabschnitte aufweisen kann. Das Schlauchführungselement 10 dient zur Anbringung und Führung von Schlauchabschnitten vorzugsweise an der Frontseite des Geräts.

An der Vorderseite des Grundkörpers 22 sind zwei Klemmbacken 20 und 21 ausgeformt, die jeweils eine Außenwölbung 23 und 24 aufweisen. Die Klemmbacken 20, 21 sind so zueinander ausgerichtet, dass die Außenwölbungen 23, 24 einander zugewandt sind und sich zwischen den Klemmbacken 20, 21 ein Klemmspalt 30 ausbildet. Der Abstand zwischen den Klemmbacken 20, 21 ist dabei so gewählt, dass ein Schlauch von vorne in den Spalt 30 eindrückbar ist, so dass der Schlauch in dem Klemmspalt 30 gehalten und geführt ist. In dem dargestellten Ausführungsbeispiel verläuft der Klemmspalt 30 vertikal, er kann jedoch auch in anderen Winkel zur Horizontalen verlaufen, wenn dies für eine Therapieart vorteilhaft sein sollte.

Die Klemmbacken 20, 21 und der Grundkörper 22 können einteilig ausgeführt sein, und aus Kunststoff bestehen. Es kann jedoch auch vorgesehen sein, dass es sich bei dem Klemmbacken 20, 21 um getrennte Einzelteile handelt, die beispielsweise auf den Grundkörper 22 aufgeklebt oder angeschraubt sind. Ferner kann das Schlauchführungselement 10 auch direkt aus dem Gehäuse 40 des medizinischen Geräts heraus ausgeformt sein.

Der Seitenansicht der Fig. 2 ist zu entnehmen, wie der Grundkörper 22 mit seiner Rückseite an einem Gehäuse 40 eines medizinischen Geräts angebracht ist. An der Vorderseite des Grundkörpers 22 befinden sich dann die Klemmbacken, wobei diese Seitenansicht in Fig. 1 einer Sicht von rechts auf das Schlauchführungselement 10 entspricht, so dass die Klemmbacke 21 zu sehen ist. Die Klemmbacken 20, 21 ragen dabei vorzugsweise an der oberen Seite über den Grundkörper 22 hinaus. Sie können auch an der unteren Seite über den Grundkörper 22 hinausragen, aber dies ist nicht zwingend erforderlich.

Durch das Überstehen der Klemmbacken 20, 21 an der oberen Seite wird zwischen den Klemmbacken 20, 21 und dem Gehäuse 40 bereits eine Führungsrinne gebildet, in welche ein Schlauch einlegbar ist. Ergänzt wird diese Rinnenbegrenzung jedoch durch eine rinnenförmige Vertiefung 31 in der oberen Seitenfläche des Grundkörpers 22. In diese Rinne 31 ist ein Schlauch 50' einlegbar, ohne dass er nach vorne über die Klemmbacken 20, 21 rutschen kann.

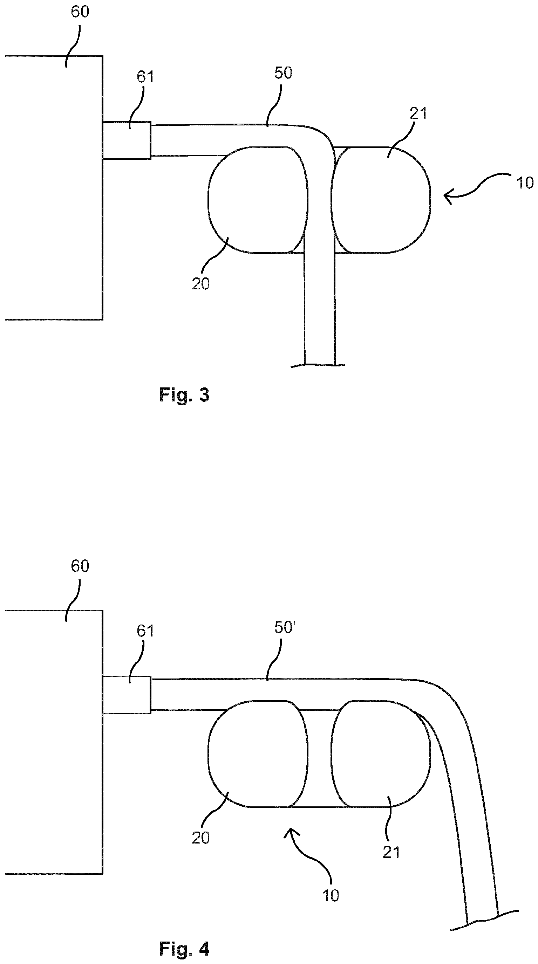

Fig. 3 zeigt einen ersten Verlegeweg für einen Schlauch 50, der durch das erfindungsgemäße Schlauchführungselement 10 vorgegeben werden kann. Dabei ist das Schlauchführungselement 10 beispielsweise an der Frontseite des Gehäuses neben einem Funktionsteil 60 des medizinischen Geräts angebracht. Bei dem Funktionsteil 60 kann es sich beispielsweise um eine Pumpe handeln, mit welcher Blut durch den Einmalartikel und damit durch einen Schlauchabschnitt gepumpt wird. Das Funktionsteil 60 weist einen Schlauchanschluss 61 auf, an welchen der Schlauch 50 angeschlossen ist. Der Schlauch 50 kann jedoch auch durch das Funktionsteil 60 hindurch verlaufen und lediglich im Bereich des Schlauchanschlusses 61 aus dem Funktionsteil 60 herausgeführt sein.

Das Schlauchführungselement 10 ist so neben dem Funktionsteil 60 angeordnet, dass die Oberseite des Grundkörpers 22 in etwa auf Höhe des Schlauchanschlusses 61 liegt, so dass der horizontal aus dem Funktionsteil 60 herausgeführte Schlauch 50 auf dem Schlauchführungselement 10 aufliegen kann, ohne dass er zwischen dem Funktionsteil 60 und dem Schlauchführungselement 10 gebogen werden muss. Für einen ersten Verlegeweg des Schlauchs 50 kann es jedoch erforderlich sein, dass der Schlauch 50 nach Verlassen des Funktionsteils 60 vertikal nach unten zu anderen Bauteilen des Geräts geführt werden muss. Daher liegt er nur teilweise auf dem Schlauchführungselement 10 auf und wird dann in der Mitte des Schlauchführungselements 10 vertikal nach unten geführt, indem der Schlauch 50 von vorne in den Klemmspalt 30 eingedrückt wurde. Ein Abknicken des Schlauchs 50 durch Zugbelastung kann hierbei verhindert werden, da die Klemmbacke 20 so ausgeformt ist, dass der Schlauch an einer abgerundeten Kante der Außenwölbung 23 umgebogen wird. Selbst wenn eine Zugbelastung den Schlauch nach unten zieht, unterschreitet der Biegeradius nicht den minimalen Biegeradius.

Fig. 4 zeigt einen zweiten Verlegeweg für einen Schlauch 50', bei welchem der Schlauch 50' ebenfalls horizontal aus dem Funktionsteil 60 herausgeführt ist und vollständig auf der Oberseite des Grundkörpers 22 aufliegt. Dabei kann wenigstens durch die oberen Kanten der Klemmbacken 20, 21 verhindert werden, dass der Schlauch 50' nach vorne herunter rutscht. Diese Haltewirkung der Klemmbacken 20, 21 kann durch die Führungsrinne 31 unterstützt werden.

Ferner ist wenigstens die rechte obere Kante des Grundkörpers 22 abgerundet ausgeführt, so dass der Schlauch 50' rechts an dem Schlauchführungselement 10 herunterhängen kann, ohne dass der Schlauch 50 an dieser Kante durch eine Zugbelastung abknicken kann. Der Radius der abgerundeten Kante ist entsprechend groß gewählt, und die abgerundete Kante geht vorzugsweise weich in die Führungsrinne 31 über.

Der erste Verlegeweg der Fig. 3 kann beispielsweise dadurch erforderlich sein, dass bei einer ersten Therapieart rechts unterhalb der Pumpe 60 ein Filter angebracht ist, an welchem der Schlauch 50 links vorbeigeführt werden muss. Bei einer zweiten Therapieart ist an dieser Stelle kein Filter angebracht, so dass der Schlauch rechts an dem Schlauchführungselement heruntergeführt werden kann.

Die Erfindung betrifft ein Schlauchführungselement (10) zur Anbringung und Führung eines Schlauchs (50;50') am Gehäuse (40) eines medizinischen Geräts zur extrakorporalen Blutbehandlung. Erfindungsgemäß weist das Schlauchführungselement (10) einen plattenförmigen Grundkörper (22) auf, der über seine Rückseite an dem Gehäuse (40) anbringbar ist. Auf der Vorderseite des Grundkörpers (22) zwei Klemmbacken (20;21) mit jeweils einer Außenwölbung (23;24) so angebracht, dass die Außenwölbungen (23;24) einander zugewandt sind und zwischen den Außenwölbungen (23;24) ein Klemmspalt (30) ausgebildet ist, in welchen ein Schlauch (50;50') einklemmbar ist. Der Grundkörper (22) weist ferner an einer oberen Seitenfläche eine Führungsrinne (31) auf, in welche ein Schlauch (50;50') einlegbar ist, und die Kante zwischen der oberen Seitenfläche und wenigstens einer weiteren Seitenfläche des Grundkörpers (22) ist abgerundet ausgeführt.

Die Erfindung betrifft ferner ein medizinisches Gerät zur extrakorporalen Blutbehandlung mit einem solchen Schlauchführungselement (10).

### Bezugszeichenliste

- 10: Schlauchführungselement
- 20,21: Klemmbacke, Führungsbacke
- 22: Grundkörper
- 23,24: Außenwölbung
- 30: Klemmspalt, Führungsspalt
- 31: Führungsrinne, Vertiefung
- 40: Gehäuse, medizinisches Gerät
- 50,50': Schlauch
- 60: Funktionsteil, Pumpe
- 61: Schlauchanschluss

## Patentansprüche

1. Schlauchführungselement (10), das dafür angepasst ist, einen Schlauch (50;50') am Gehäuse (40) eines medizinischen Geräts zur extrakorporalen Blutbehandlung anzubringen und zu führen, wobei das Schlauchführungselement (10) einen Grundkörper (22) aufweist, der über eine erste Grundkörperseite an dem Gehäuse (40) anbringbar ist, und **dadurch gekennzeichnet, dass** auf einer zweiten, vorzugsweise der ersten Grundkörperseite, abgewandten Seite des Grundkörpers (22) zwei Klemmbacken (20;21) mit jeweils einer Außenwölbung (23;24) so angebracht sind, dass die Außenwölbungen (23;24) einander zugewandt sind und zwischen den Außenwölbungen (23;24) ein Klemmspalt (30) ausgebildet ist, in welchen ein Schlauch (50;50') einklemmbar ist, und dass der Grundkörper (22) auf einer dritten Grundkörperseite eine Führungsrinne (31) aufweist, in welche ein Schlauch (50;50') einlegbar ist, wobei die Kante zwischen der dritten Grundkörperseite und wenigstens einer weiteren Seite des Grundkörpers (22) abgerundet ausgeführt ist.

2. Schlauchführungselement nach Anspruch 1,
**dadurch gekennzeichnet, dass** die Führungsrinne (31) wenigstens durch eine Vertiefung in der dritten Seite des Grundkörpers (22) gebildet ist.

3. Schlauchführungselement nach einem oder beiden der Ansprüche 1 und 2,
**dadurch gekennzeichnet, dass** die Führungsrinne (31) wenigstens durch die Klemmbacken (20;21) gebildet ist, indem die Klemmbacken (20;21) über die dritte Seite des Grundkörpers (22) hinausragen.

4. Schlauchführungselement nach einem oder mehreren der Ansprüche 1 bis 3,
**dadurch gekennzeichnet, dass** die Vertiefung (31) weich in die abgerundete Kante der dritten Seite übergeht.

5. Schlauchführungselement nach einem oder mehreren der Ansprüche 1 bis 3,
**dadurch gekennzeichnet, dass** der Klemmspalt (30) quer zur Führungsrinne (31) verläuft.

6. Schlauchführungselement nach Anspruch 5,
**dadurch gekennzeichnet, dass** der Klemmspalt (30) senkrecht zur Führungsrinne (31) verläuft.

7. Medizinisches Gerät zur extrakorporalen Blutbehandlung, umfassend ein Gehäuse (40) und ein Schlauchführungselement (10), das an einer Seite des Gehäuses (40) angebracht ist,
**dadurch gekennzeichnet, dass** das Schlauchführungselement (10) gemäß einem oder mehreren der Ansprüche 1 bis 6 ausgebildet ist.

8. Medizinisches Gerät nach Anspruch 7,
**dadurch gekennzeichnet, dass** das Schlauchführungselement (10) so ausgebildet und am Gehäuse (40) angebracht ist, dass der Klemmspalt (30) vertikal verläuft, während die Führungsrinne (31) an der dritten, nunmehr oberen Seite des Grundkörpers (22) horizontal verläuft.

9. Medizinisches Gerät nach einem oder beiden der Ansprüche 7 und 8,
**dadurch gekennzeichnet, dass** mit dem medizinischen Gerät wenigstens zwei unterschiedliche Therapiearten durchführbar sind, welche verschiedene Verlegewege eines Schlauchs (50;50') vorsehen, und dass der Schlauch (50) bei einem ersten Verlegeweg durch den Klemmspalt (30) zu führen ist, während der Schlauch (50') für den zweiten Verlegeweg in die Führungsrinne (31) einzulegen ist.

10. Medizinisches Gerät nach Anspruch 9,
**dadurch gekennzeichnet, dass** das Schlauchführungselement (10) neben einem Funktionsteil (60) des medizinischen Geräts angebracht ist, aus dem ein Schlauch (50;50') horizontal aus einem Schlauchanschluss (61) herausgeführt ist, und das Schlauchführungselement (10) so neben diesem Funktionsteil (60) angeordnet ist, dass die obere Seitenfläche des Grundkörpers (22) auf Höhe des Schlauchanschlusses (61) liegt, und der Schlauch (50) in dem ersten Verlegeweg teilweise in der Führungsrinne (31) aufliegt und dann durch den Klemmspalt (30) nach unten geführt ist, während der Schlauch (50') in dem zweiten Verlegeweg vollständig in der Führungsrinne (31) aufliegt und über die abgerundete Kante der oberen Seite nach unten hängt.

## Claims

1. Tube guidance element (10) which is configured to attach and guide a tube (50;50') on the housing (40) of a medical device for extracorporeal blood treatment wherein the tube guidance element (10) has a base body (22), which is attachable to the housing (40) via a first base body side, and **characterized in that**
two clamping jaws (20; 21) each having an outward bulge (23; 24) are mounted on a second side of the base body (22), preferably facing away from the first base body side, in such a way that the outward bulges (23; 24) are facing each other and that a clamping gap (30) is formed between the outward bulges (23; 24), into which a tube (50; 50') can be clamped, and that the base body (22) has a guide channel (31) on a third base body side into which a tube (50; 50') can be inserted, wherein the edge between the third base body side and at least one further side of the base body (22) has a rounded shape.

2. Tube guidance element according to claim 1,
**characterized in that** the guide channel (31) is formed at least by a recess in the third side of the base body (22).

3. Tube guidance element according to one or both of claims 1 and 2,
**characterized in that** the guide channel (31) is formed at least by the clamping jaws (20; 21) in the way that the clamping jaws (20; 21) extend beyond the third side of the base body (22).

4. Tube guidance element according to one or more of claims 1 to 3,
**characterized in that** the recess (31) transitions smoothly into the rounded edge of the third side.

5. Tube guidance element according to one or more of claims 1 to 3, **characterized in that** the clamping gap (30) runs transversely to the guide channel (31).

6. Tube guidance element according to claim 5,
**characterized in that** the clamping gap (30) runs perpendicularly to the guide channel (31).

7. Medical device for extracorporeal blood treatment, comprising a housing (40) and a tube guidance element (10) attached to a side of the housing (40),
**characterized in that**
the tube guidance element (10) is designed according to one or more of claims 1 to 6.

8. Medical device according to claim 7,
**characterized in that** the tube guidance element (10) is constructed and attached to the housing (40) in such a way that the clamping gap (30) extends vertically, while the guide channel (31) extends horizontally on the third, now upper side of the base body (22).

9. Medical device according to one or both of claims 7 and 8
**characterized in that**, at least two different types of therapy can be performed with the medical device which use different installation paths of a tube (50; 50'), and that the tube (50) is to be guided through the clamping gap (30) in a first installation path, while the tube (50') is to be inserted into the guide channel (31) for the second installation path.

10. Medical device according to claim 9,
**characterized in that** the tube guidance element (10) is mounted next to a functional part (60) of the medical device from which a tube (50; 50') is guided horizontally out of a tube connection (61), and the tube guidance element (10) is arranged in such a way next to this functional part (60) that the upper side surface of the base body (22) is at the level of the tube connection (61), and the tube (50) in the first installation path partially rests in the guide channel (31) and is then guided downward through the clamping gap (30), while the tube (50') in the second installation path fully rests in the guide channel (31) and hangs down over the rounded edge of the upper side.

## Revendications

1. Élément de guidage de tuyau (10), conçu pour monter et guider un tuyau (50 ; 50') sur le boîtier (40) d'un appareil médical pour le traitement extracorporel du sang,
l'élément de guidage de tuyau (10) comprenant un corps de base (22) qui peut être monté sur le boîtier (40) au-dessus d'un premier côté du corps de base et **caractérisé en ce que**, sur un deuxième côté du corps de base (22), de préférence opposé au premier côté du corps de base, des mâchoires de serrage (20 ; 21) avec chacune une courbure externe (23 ; 24) sont montées de façon à ce que les courbures externes (23 ; 24) soient orientées l'une vers l'autre et, entre les courbures externes (23 ; 24), un interstice de serrage (30) soit formé, dans lequel un tuyau (50 ; 50') peut être inséré et **en ce que** le corps de base (22) comprend, sur un troisième côté du corps de base, une rainure de guidage (31) dans laquelle un tuyau (50 ; 50') peut être inséré, l'arête entre le troisième côté du corps de base et au moins un autre côté du corps de base (22) étant réalisée de manière arrondie.

2. Élément de guidage de tuyau selon la revendication 1,
**caractérisé en ce que** la rainure de guidage (31) est constitué au moins d'une cavité dans le troisième côté du corps de base (22).

3. Élément de guidage de tuyau selon l'une ou les deux revendications 1 et 2,
**caractérisé en ce que** la rainure de guidage (31) est constituée au moins des mâchoires de serrage (20 ; 21) grâce au fait que les mâchoires de serrage (20 ; 21) dépassent du troisième côté du corps de base (22).

4. Élément de guidage de tuyau selon l'une ou plusieurs revendications 1 à 3,
**caractérisé en ce que** la cavité (31) se transforme progressivement en l'arête arrondie du troisième côté.

5. Élément de guidage de tuyau selon l'une ou plusieurs revendications 1 à 3,
**caractérisé en ce que** l'interstice de serrage (30) s'étend transversalement par rapport à la rainure de guidage (31).

6. Élément de guidage de tuyau selon la revendication 5,
**caractérisé en ce que** l'interstice de serrage (30) s'étend perpendiculairement par rapport à la rainure de guidage (31).

7. Appareil médical pour le traitement extracorporel du sang, comprenant un boîtier (40) et un élément de guidage de tuyau (10), qui est monté sur un côté du boîtier (40),
**caractérisé en ce que** l'élément de guidage de tuyau (10) est conçu selon l'une ou plusieurs des revendications 1 à 6.

8. Appareil médical selon la revendication 7,
**caractérisé en ce que** l'élément de guidage de tuyau (10) est conçu et monté sur le boîtier (40) de façon à ce que l'interstice de serrage (30) s'étende verticalement, tandis que la rainure de guidage (31) s'étend horizontalement le long du troisième côté, désormais supérieur, du corps de base (22).

9. Appareil médical selon l'une ou les deux revendications 7 et 8,
**caractérisé en ce que**, avec l'appareil médical, au moins deux types de traitements peuvent être effectués, qui prévoient différentes voies de pose d'un tuyau (50 ; 50') et **en ce que** le tuyau (50) doit être guidé, pour une première voie de pose, à travers l'interstice de serrage (30), tandis que le tuyau (50') pour la deuxième voie de pose doit être posé dans la rainure de guidage (31).

10. Appareil médical selon la revendication 9,
**caractérisé en ce que** l'élément de guidage de tuyau (10) est monté à proximité d'une partie fonctionnelle (60) de l'appareil médical, à partir de laquelle un tuyau (50 ; 50') est guidé horizontalement à partir d'un raccord de tuyau (61) et l'élément de guidage de tuyau (10) est disposé à proximité de cette partie fonctionnelle (60) de façon à ce que la face latérale supérieure du corps de base (22) se trouve à la hauteur du raccord de tuyau (61) et le tuyau (50) dans la première voie de pose est posé partiellement dans la rainure de guidage (31) puis est guidé vers le bas à travers l'interstice de serrage (30), tandis que le tuyau (50') dans la deuxième voie de pose est posé entièrement dans la rainure de guidage (31) et est suspendu vers le bas au-dessus de l'arête arrondie du côté supérieur.
